# EUROPEAN PATENT APPLICATION

(11) **EP 1 684 203 A2**
(43) Date of publication of application: **26.07.2006**
(21) Application number: 05012244.9
(22) Date of filing: 07.06.2005
(51) Int. Cl.: G06F 19/00

(54) **Pharmaceutical automation management system**

(30) Priority: 25.01.2005 KR 2005006828
(71) Applicant: JVM Co., Ltd., Daegu 704-170 (KR)
(72) Inventor: Kim, Jun-ho, Dalseo-Gu Daegu, 704-923 (KR)
(74) Representative: Grünecker, Kinkeldey, Stockmair & Schwanhäusser Anwaltssozietät

(57) **Abstract**

A pharmaceutical automation management system is disclosed. The administrator authentication module authenticates an administrator and registers a login time of the authenticated administrator in a recording medium. The user interface module displays a user interface screen and inputs data through option items which can be selected in the user interface screen. The drug information registration module registers tablet information based on the tablet cassettes inputted through the option items in a recording medium. The a server connection module connects to at least one or more prescription providing servers whose paths are designated through the option items, accessing prescription files, and registering the prescription files in the recording medium. The prescription transmission module interfaces the prescription files registered in the recording medium with designated tablet packaging machines through the option item.

## Description

### BACKGROUND OF THE INVENTION

### Field of the Invention

The present invention relates to a pharmaceutical automation management system, and more particularly to a system for automating and managing tasks in a dispensary or drugstore.

### Description of the Related Art

A pharmaceutical automation management system is a system which enables a tablet automatic packaging machine in a dispensary or a drugstore, such as an automatic tablet dispensing and packaging system, etc., to automatically perform all pharmaceutical tasks, such as, selection, classification, determination of the number of prescribed tablets, dispensation of tablets, automatic packaging and printing information for taking tablets, etc., on the basis of prescription, when prescription data for a patient are inputted to the computer which is connected to the tablet automatic packaging machine via a network.

As prescription tasks are automated, the patient does not need to wait for his prescription for a relatively long time, and a pharmacist can faithfully inform a tablet taking method of the patient, thereby enhancing competitiveness of a dispensary or a drugstore. Also, since personal expenses, etc., can be reduced while a pharmacist's productivity is increased, income of the dispensary or drugstore can be improved. In addition, such a system can prevent a careless prescription and medication errors.

However, the prior art systems have been designed such that they can simply and automatically perform a series of prescription steps based on a prescription, but do not support tasks necessary for a pharmacist's efficient working processes, such as drug management, tracing and referring a refiller who refills the ATDPS with tablets.

Therefore, a novel pharmaceutical automation management system for efficiently managing a dispensary or drugstore and for improving pharmacist's work efficiency is required.

### SUMMARY OF THE INVENTION

Therefore, the present invention has been made in view of the above problems, and it is an object of the present invention to provide a pharmaceutical automation management system which is capable of automatically managing expiration dates of tablets contained in a tablet packaging machine such as an automatic tablet dispensing and packaging system (ATDPS), etc., and easily referring to a manager who refills the tablet packaging machine with tablets.

It is another object of the present invention to provide a pharmaceutical automation management system which is capable of printing various data based on a type of report, in which the various data is used for total management of tablets contained in a tablet packaging machine.

It is a further object of the present invention to provide a pharmaceutical automation management system which is capable of automatically classifying prescriptions based on wards, and designating tablet packaging machines located at every ward, such that the prescriptions base on ward are automatically interfaced with corresponding tablet packing machines.

It is still another object of the present invention to provide a pharmaceutical automation management system which is capable of editing a printing format for a tablet pouch based on wards.

It is a still further object of the present invention to provide a pharmaceutical automation management system which is capable of automatically registering tablet information and prescription data, etc.

It is yet another object of the present invention to provide a recording medium for recording program data which is necessary for easily constructing such pharmaceutical automation management systems in users' computers, in which the program data is readable by the computers.

In accordance with the present invention, the above and other objects can be accomplished by the provision of a pharmaceutical automation management system comprising: an administrator authentication module for authenticating an administrator and registering a login time of the authenticated administrator in a recording medium; a user interface module for displaying a user interface screen and inputting data through option items which can be selected in the user interface screen; a drug information registration module for registering tablet information based on the tablet cassettes inputted through the option items in a recording medium; a server connection module for connecting to at least one or more prescription providing servers whose paths are designated through the option items, accessing prescription files, and registering the prescription files in the recording medium; and a prescription transmission module interfacing the prescription files registered in the recording medium with designated tablet packaging machines through the option item.

Preferably, the system may further comprise a pouch layout setting module for variably setting positions of data to be printed in pouches, in which the data is inputted through the option screen of the user interface screen.

Preferably, the system may further comprise a prescription automatic classification module for automatically classifying prescription files based on wards, in which the prescription files are registered in the recording medium.

Preferably, the system may further comprise a report generation module for generating and outputting one of a refill cassette report, a shake report, a refill cancel report, an administrator login report, and a special tablet system (STS) fill report, according to a report generation command which can be selected through an option screen of the user interface screen, based on registered data.

In accordance with another aspect of the present invention, there is provided a recording medium for recording program data which is necessary for constructing the pharmaceutical automation management system.

### BRIEF DESCRIPTION OF THE DRAWARDS

The above and other objects, features and other advantages of the present invention will be more clearly understood from the followard detailed description taken in conjunction with the accompanying drawards, in which:
Fig. 1 is a view illustrating a pharmaceutical automation management system according to an embodiment of the present invention, and peripherals thereof;
Fig. 2 is a detailed view illustrating modules of the pharmaceutical automation management system according to an embodiment of the present invention, which is constructed in a computer system; and
Fig. 3 is a view illustrating a user interface for variably setting positions of information to be printed on a pouch according to an embodiment of the present invention.

### DESCRIPTION OF THE PREFERRED EMBODIMENTS

Now, preferred embodiments of the present invention will be described in detail with reference to the annexed drawards. In the followard description, a detailed description of known functions and configurations incorporated herein will be omitted when it may make the subject matter of the present invention rather unclear.

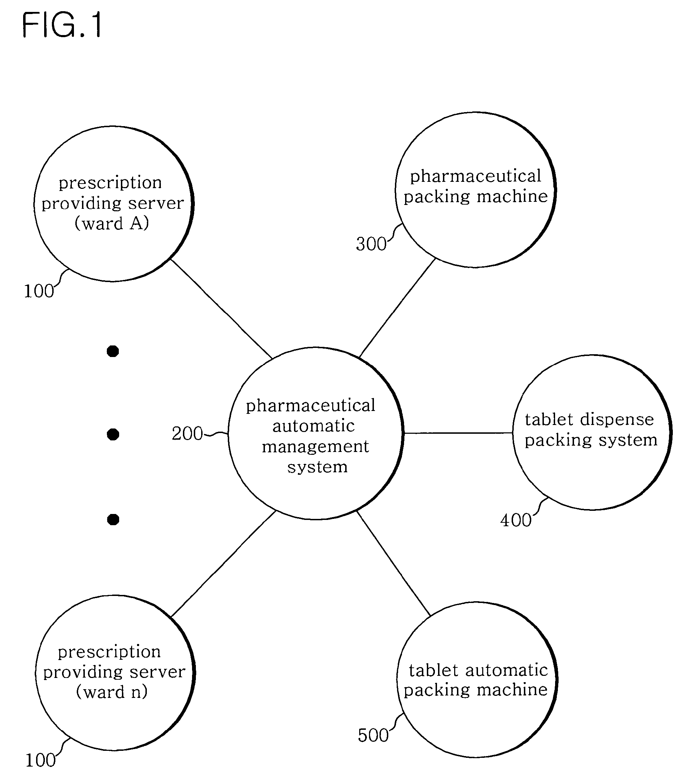

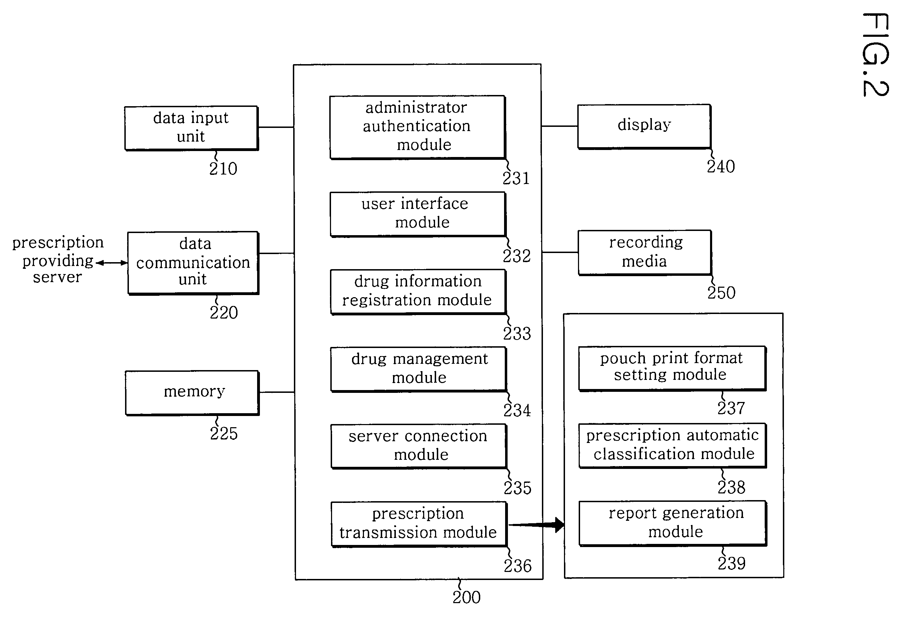

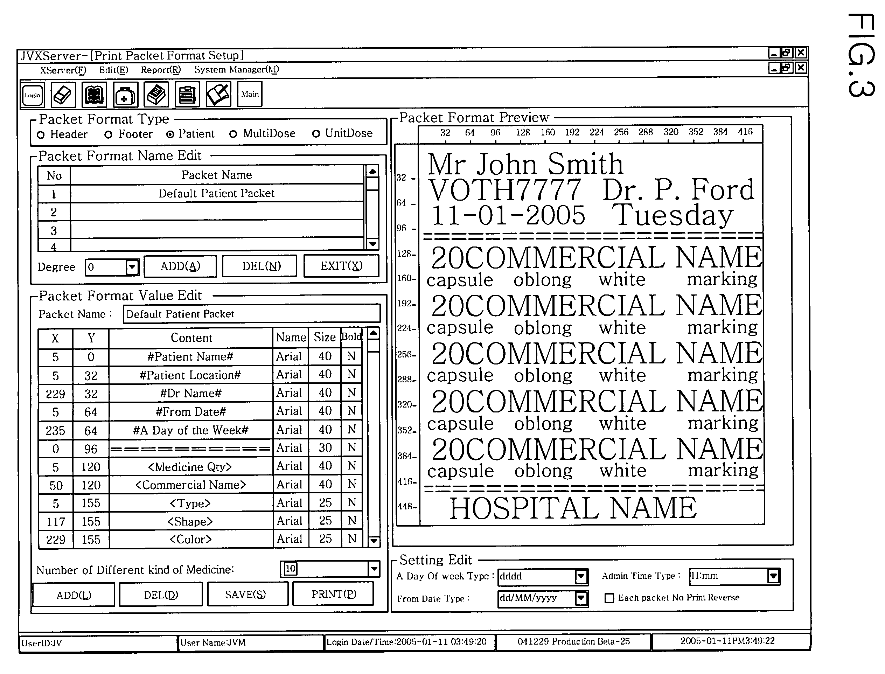

Firstly, Fig. 1 is a view illustrating a pharmaceutical automation management system 200 according to an embodiment of the present invention, and peripherals thereof. Fig. 2 is a detailed view illustrating modules of the pharmaceutical automation management system 200 according to an embodiment of the present invention, which is constructed in a computer system. Fig. 3 is a view illustrating a user interface for variably setting positions of information to be printed on a pouch according to an embodiment of the present invention.

Referring to Fig. 1, the pharmaceutical automation management system 200 is installed on a computer system in a dispensary or a drugstore and then connected to at least one or more prescription-providing servers 100 via a network.

The prescription-providing server 100 is implemented with a server located at each ward in a hospital, or a server located at each hospital in a local area according to circumstances. Such a prescription-providing server 100 basically provides prescription files for patients, files of tablet information and files of tablet taking method. File providing methods are implemented such that, as a folder of the server 100 is previously designated as a file providing folder, the pharmaceutical automation management system 200 periodically accesses the designated folder to fetch new files stored therein.

On the other hand, the pharmaceutical automation management system 200 installed and operated in the computer system is a collection of program modules capable of being readout by a main control unit of the computer system. Namely, as shown in Fig. 2, the pharmaceutical automation management system 200 includes a plurality of modules and is distributed through magnetic recording media or optical recording media, which are publicly well known.

Referring to Fig. 2, the pharmaceutical automation management system 200 includes an administrator authentication module which authenticates an administrator and registers a login time of the authenticated administrator in a recording medium 250. Such an administrator authentication module is uses to easily refer to an administrator of the pharmaceutical automation management system 200, in which the administrator is referred to a person who prepares tablets based on a prescription related to medication errors.

On the other hand, as shown in Fig. 3, the system 200 includes a user interface module 232 which displays a user interface screen on a display 240 of a computer system, inputs data through selectable option items in the screen, and transmits the data to corresponding program modules which will be described later. Such a user interface module 232 enables an administrator to select functions, such as necessary drug information registration, login administrator registration, report creation and output, etc.

The system 200 includes a drug information registration module which registers drug information based on cassettes in a recording medium 250, in which the drug information is inputted thereto as the option items located at the upper part of the user interface screen of Fig. 3 are selected. Here, an input window to which drug information is inputted includes fields, such as the cassette number of the tablet packaging machine, drug code, drug name, expiration date, medicine barcode, quantity (number of tablets), etc., such that an administrator can easily input corresponding information thereto. Here, the expiration date denotes a term counted from the date of drug manufacture or from the date of drug refilling. If the expiration date is a former case, it requires a manual update. On the other hand, if the expiration date is a latter case, it can be automatically updated. When tablet refilling is performed, drug refill time, refill quantity, an expiration date, a tablet refiller, etc., are updated by the drug information registration module 233.

The system 200 includes a drug management module 234 which requires information of tablets which expire their expiration date, as a piece of tablet information based on tablet cassettes which are registered in the recording medium 250, through the user interface screen. Namely, such information of tablets which expire their expiration date is displayed on a display by the drug management module 234, such that an administrator can view the table information. When a psychotropic medicine and general medicines are refilled, the drug management module 234 may be required to output their current quantity through the user interface screen. Also, the drug management module 234 allows an administrator to edit tablet information according to tablets.

The system 200 basically includes a server connection module 235 which connects to at least one or more prescription providing servers 100 whose paths are designated through the option screen, accesses prescription files, and registers the prescription files in the recording medium 250. Namely, such a server connection module 235 connects to at lease one or more prescription providing servers 100 whose paths are designated, accesses files of tablet taking method and drug information files, and adds and registers the files in the recording medium 250.

The system 200 basically includes a prescription transmission module 236 which interfaces the prescription files registered in the recording medium 250 with designated tablet packaging machines 300, 400, and 500 through the option item. Such a prescription transmission module 236 enables an administrator to match the prescription providing servers 100 based on wards with the tablet packaging machines 300, 400, and 500, in a one to one correspondence, thereby preparing corresponding tablets.

Also, the system 200 includes a pouch layout setting module 237, a prescription automatic dispenser module 238, and a report generation module 239.

The pouch layout setting module 237 variably sets a position of data to be printed, in which the data is inputted through the option screen of the user interface screen. More specifically, the pouch layout setting module 237 variably sets the position of data to be printed according to position coordinate data (X, Y) depending on contents. Such a pouch layout setting module 237 allows print patterns for pouches to be differently set depending on wards.

On the other hand, the prescription automatic classification module 238 automatically classifies prescription files which are registered in the recording medium 250 according to user commands, in which the user commands can be selected through an option screen of the user interface screen. Namely, such a prescription automatic classification module 238 enables an administrator to firstly proceed all the prescriptions of a ward and then proceed prescriptions of a next ward.

The report generation module 239 generates and outputs one of a refill cassette report, a shake report, a refill cancel report, an administrator login report, and a special tablet system (STS) fill report, according to a report generation command which can be selected through an option screen of the user interface screen, based on data registered in the recording medium 250. Such a report generation module 239 allows an administrator to easily view information, etc., which is related to preparation tasks, for example, login administrator information, operation state information of tablet packaging machines, current remaining quantity information, information of drug quantity for a prescription.

Procedures for setting program preference and for performing preparation according to prescriptions, after such a pharmaceutical automation management system 200 is installed on a computer system, are described in detail below.
1) Firstly, an administrator in a dispensary or a drugstore installs the pharmaceutical automation management system 200 on a computer system, in which the pharmaceutical automation management system 200 is a collection of program modules readable in a main control unit of a computer system.
2) After that, the pharmaceutical automation management system 200 is executed on the computer system. The administrator authentication module 231 requires authentication information for authenticating an administrator.
3) The administrator inputs his/her identification (ID) and password to the administrator authentication module 231 to register administrator authentication information. Such administrator authentication information allows authentication information of a plurality of administrators to collectively register thereto when the pharmaceutical automation management system is installed. On the other hand, an authenticated person can periodically update the administrator authentication information if necessary.
4) When registration of the administrator authentication information is completed, as shown in Fig. 3, the user interface module 232 displays a user interface screen on a display. An administrator can set program preferences through system manager option items of the user interface screen. Namely, the administrator can set the number and the types of tablet packaging machines 200, 400, and 500, which are connected to the pharmaceutical automation management system 200, IP number of the network, port numbers, etc. Also, destination information for periodically accessing prescription files or files for tablet taking methods, drug information files, etc., is set. Namely, shared folders of a prescription providing server 100 are set as a destination drive.
5) On the other hand, an administrator can set the tablet packaging machine through edit option items of the user interface screen, on a hospital of hospital-ward basis.
6) Drug information and information of tablet taking method are set to be manually or automatically registered through option items of the user interface screen. According to the setting conditions, the drug information registration module 233 registers information of tablets contained in cassettes in a recording medium 250, in which the information of tablets is inputted thereto by an administrator. Also, the server connection module 235 connects to the prescription providing server 100 whose path has been designated, accesses files of tablet taking method and files of tablet information, and registers the files in the recording medium 250.
7) A layout to be printed on pouches is set through option item of the user interface screen. Using such a function, layouts can be differently set based on wards.
8) When the program preferences are set and registered, the server connection module 235 periodically accesses the prescription-providing server 100 whose path has been designated and registers files of new prescriptions in the recording medium 250.
9) After that, the prescription transmission module 236 transmits corresponding prescription files to designated tablet packaging machines 300, 400, and 500; thereby preparing tablets based on the registered prescription files.

Here, each of steps 4) to 7) can be selectively performed regardless of their sequences.

Now, operation of the pharmaceutical automation management system 200 whose program preferences are set according to the above-mentioned procedures is described in detail below.

### - Administrator Reference

The pharmaceutical automation management system 200 according to an embodiment of the present invention requires an administrator login/password. Namely, an administrator working at a dispensary or a drugstore should log in/log out of the system 200 starting/ending with his/her working hours. The administrator authentication module 231 registers login time of an administrator in the recording medium 250, such that an administrator login report can be created later.

### - Automatic management of a expiration date of loaded drugs and tablet refller reference

An administrator logged in to the pharmaceutical automation management system 200 can input cassette numbers of the tablet packaging machines 300, 400, and 500, drug codes, drug names, a expiration date, medicine barcodes, and quantity (the number of tablets) thereto. Such drug information can be registered in the recording medium 250 through the drug information registration module 233.

Therefore, the drug management module 234 reads out information of tablets whose expiration dates have passed, as a piece of tablet information based on tablet cassettes which are registered in the recording medium 250, and requires the recording medium 250 to output the information through the user interface screen. Then, the administrator can recognize the information of tablets whose expiration dates have passed, through the user interface screen. As a result, the expiration date of tablets loaded in the tablet packaging machine can be automatically managed.

After that, the administrator refills, or exchanges the expired tablets in corresponding tablet cassettes. Here, the drug information registration module 233 updates drug refill information, such that the administrator on duty in a drug refill time can be referred or a table refiller can be referred based on a refill cassette report.

### - Tablet pouch print pattern setting on ward basis

An administrator logged in to the system 200 can change and set a layout for pouches through the option screen of the user interface screen. Namely, as shown in Fig. 3, when position coordinate data (X, Y) are differently set based on each content and wards through the user interface screen, data to be printed based on wards are transmitted to the tablet packaging machines 300, 400, and 500, respectively. Therefore, the tablet pouches outputted from the tablet packaging machines 300, 400, and 500 have unique layouts corresponding to each ward.

### - Prescription automatic classification

The server connection module 235 of the system 200 periodically connects to the prescription providing server 100 whose path has been designated, accesses the prescription files, and registers them in the recording files 250. The registered prescription files are automatically classified according to prescription automatic classification commands of the administrator depending on wards. Also, the classified prescription files are collectively transmitted to the tablet packaging machines 300, 400, and 500, which are previously classified based on wards, such that the tablet packaging machines 300, 400, and 500 can rapidly perform a series of preparations.

### - Update of files of tablet taking methods and drug information files

On the other hand, the server connection module 235 periodically connects to the prescription providing servers 100 whose paths are designated, accesses corresponding files if there are files of tablet taking method and tablet information files, and registers the files in the recording medium 250. Therefore, a database of the prescription providing server 100 is consistent with that of the pharmaceutical automation management system 100, although an administrator does not intervene.

### - Report output

AS information of an administrator logged in to the system 200 is registered therein by the administrator authentication module 231, the report generation module 239 can output an administrator login report based on the registered information.

Also, in the case that the cassettes have been refilled, as information of tablets is registered by the drug information registration module 233, the report generation module 239 can output cassette refill reports.

The report generation module 239 may output a record for the number of retries based on a Shake Report, in which the retries are performed since the tablets are not empty in the cassettes. Also, the report generation module 239 may output a cancelled record based on a Refill Cancel Report when tablets are refilled.

Also, the report generation module 239 may output a record for a prescription, which is prepared using a special tablet system (STS), based on an STS fill report type. When tablets are loaded in the STS, a record for operation result of the tablet packaging machine can be outputted based on an STS procedure report type.

As such, information managed and controlled by the pharmaceutical automation management system 200 must be registered in the recording medium 250.

From the operations of the embodiment of the present invention, unlike the prior art system which simply performs interface between a prescription providing server and an tablet packaging machine, since the pharmaceutical automation management system 200 according to the present invention can perform management of tablets loaded in the tablet packaging machines, management of administrators logged therein, and output of various types of reports, it can enhance efficiency and reliability of pharmacists' works.

As described above, the pharmaceutical automation management system of the present invention has advantages in that, since it automatically manages an expiration date of tablets loaded in the tablet packaging machines such as an ATDPS, etc., it can basically prevent the problems that may generated by using the tablets whose expiration dates have passed.

Also, the pharmaceutical automation management system does not need additional efforts to manage the expiration date of tablets loaded therein.

In addition, the pharmaceutical automation management system has substantial advantages in that, since an administrator who refills tablets in the system or an on duty administrator can be easily referred, culpability for medication errors can be conclusively determined.

Further, since the pharmaceutical automation management system can output various data based on a report type, in which the various data are related to an administrator logged in the system, information of tablets loaded in the tablet packaging machine, such as an ATDPS, etc., and operation states of the tablet packaging machine, the administrator can conveniently manage a dispensary or a drugstore.

Still further, the pharmaceutical automation management system can automatically classify prescriptions based on wards, collectively prepare tablets, and designate tablet packaging machines based on wards, thereby providing convenience of tablet management to administrators.

Yet further, since the pharmaceutical automation management system allow editing of layouts of tablet pouches based on wards, the pouches in which tablets are contained according to prescriptions can be easily classified.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A pharmaceutical automation management system comprising:
an administrator authentication module for authenticating an administrator and registering a login time of the authenticated administrator in a recording medium;
a user interface module for displaying a user interface screen and inputting data through option items which can be selected in the user interface screen;
a drug information registration module for registering tablet information based on the tablet cassettes inputted through the option items in a recording medium;
a server connection module for connecting to at least one or more prescription providing servers whose paths are designated through the option items, accessing prescription files, and registering the prescription files in the recording medium; and
a prescription transmission module interfacing the prescription files registered in the recording medium with designated tablet packaging machines through the option item.

2. The system as set forth in claim 1, further comprising:
a pouch layout setting module for variably setting positions of data to be printed in pouches, in which the data is inputted through the option screen of the user interface screen.

3. The system as set forth in claim 1 or 2, further comprising:
a prescription automatic classification module for automatically classifying prescription files based on wards, in which the prescription files are registered in the recording medium.

4. The system as set forth in claim 3, further comprising:
a drug management module for requiring information of tablets whose expiration dates have passed, as a piece of tablet information based on tablet cassettes which are registered in the recording medium, through the user interface screen.

5. The system as set forth in claim 4, wherein the drug management module requires to output current quantity of a psychotropic medicine and general medicines through the user interface screen, when the psychotropic medicine and the general medicines are refilled.

6. The system as set forth in claim 4, further comprising:
a report generation module for generating and outputting one of a refill cassette report, a shake report, a refill cancel report, an administrator login report, and a special tablet system (STS) fill report, according to a report generation command which can be selected through an option screen of the user interface screen, based on registered data.

7. The system as set forth in claim 4, wherein the server connection module connects to at least one or more prescription providing servers whose paths are designated through the option screen, accesses files of tablet taking method and files of tablet information, and registers the files in the recording medium.

8. The system as set forth in claim 1 or 2, further comprising:
a report generation module for generating and outputting one of a refill cassette report, a shake report, a refill cancel report, an administrator login report, and a special tablet system (STS) fill report, according to a report generation command which can be selected through an option screen of the user interface screen, based on registered data.

9. The system as set forth in claim 8, further comprising:
a drug management module for requiring information of tablets whose expiration dates have passed, as a piece of tablet information based on tablet cassettes which are registered in the recording medium, through the user interface screen.

10. The system as set forth in claim 9, wherein the server connection module connects to at least one or more prescription providing servers whose paths are designated through the option screen, accesses files of tablet taking method and files of tablet information, and registers the files to the recording medium.

11. The system as set forth in claim 2, wherein the drug management module requires to output current quantity of a psychotropic medicine and general medicines through the user interface screen, when the psychotropic medicine and the general medicines are refilled.

12. A recording medium for recording program data which is necessary for constructing the pharmaceutical automation management system as set forth in any one of claims 1 to 11.
